(19) Europäisches Patentamt
European Patent Office
Office européen des brevets

(11) Veröffentlichungsnummer: **0 430 883 A2**

(12) # EUROPÄISCHE PATENTANMELDUNG

(21) Anmeldenummer: **90810905.1**

(22) Anmeldetag: **22.11.90**

(51) Int. Cl.5: **C07D 403/12**, C07D 413/12, C07D 417/12, A61K 31/505, A01N 43/54

(30) Priorität: **01.12.89 CH 4299/89**

(43) Veröffentlichungstag der Anmeldung: **05.06.91 Patentblatt 91/23**

(84) Benannte Vertragsstaaten: **AT BE CH DE DK ES FR GB GR IT LI LU NL SE Patentblatt**

(71) Anmelder: **CIBA-GEIGY AG**
**Klybeckstrasse 141**
**CH-4002 Basel(CH)**

(72) Erfinder: **Molleyres, Louis-Pierre, Dr.**
**Walter-Fürst-Strasse 5**
**CH-4102 Binningen(CH)**
Erfinder: **Gallay, Jean-Jacques, Dr.**
**Blumenrain 19**
**CH-4312 Magden(CH)**

(54) **Anthelminthika.**

(57) Es werden neue Anthelminthika beschrieben, die als Wirkstoff eine Verbindung der Formel I

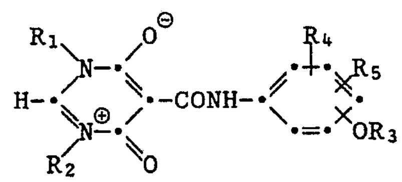

(I)

oder in ihrer hydrierten Form der Formel Ia

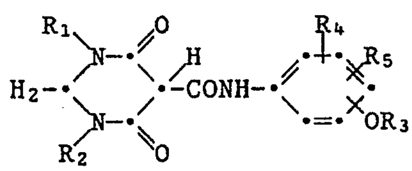

(Ia)

aufweisen, worin
$R_1$ und $R_2$ unabhängig voneinander für $C_1$-$C_6$-Alkyl, Allyl, $C_3$-$C_6$-Cycloalkyl, Benzyl oder Phenyl stehen; und $R_3$ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol; und $R_4$ und $R_5$ unabhängig voneinander Wasserstoff, $C_1$-$C_6$-Alkyl, $C_1$-$C_6$-Haloalkyl, $C_1$-$C_6$-Alkoxy oder $C_1$-$C_6$-Haloalkoxy bedeuten, unter Einschluss ihrer tautomeren Formen und physiologisch verträglichen Salze, enthalten. Es wird auch die Herstellung, Formulierung und Verwendung dieser Wirkstoffe beschrieben.

EP 0 430 883 A2

## ANTHELMINTHIKA

Die vorliegende Erfindung betrifft neue 1,3-disubstituierte 5-Azolyloxyphenyl-carbamoyl-4,6-pyrimidindion-derivate der nachstehenden Formel I und deren hydrierte Analoga der nachstehenden Formel Ia mit anthelminthischer Wirksamkeit; besagte Substanzen zur Verwendung in einem Verfahren zur Bekämpfung von Helminthen; Anthelminthische Mittel die diese Substanzen als Wirkstoffe enthalten; die Herstellung der Wirkstoffe und Mittel; sowie die Verwendung der Wirkstoffe bzw. Mittel zur Bekämpfung von Helminthen, insbesondere von Nematoden, Cestoden und Trematoden in Haus- und Nutztieren aus der Klasse der Säugetiere.

Die erfindungsgemässen Verbindungen haben die Formel I

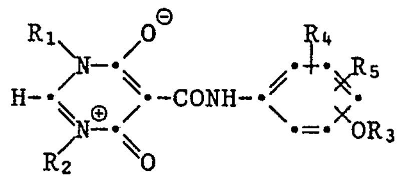

(I)

oder in ihrer hydrierten Form die Formel (Ia)

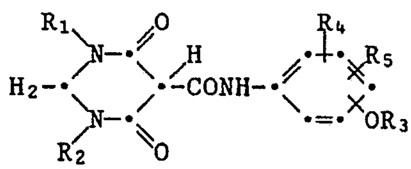

(Ia)

worin

$R_1$ und $R_2$ unabhängig voneinander für $C_1$-$C_6$-Alkyl, Allyl, $C_3$-$C_6$-Cycloalkyl, Benzyl oder Phenyl stehen; und

$R_3$ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol; und $R_4$ und $R_5$ unabhängig voneinander Wasserstoff, $C_1$-$C_6$-Alkyl, $C_1$-$C_6$-Haloalkyl, $C_1$-$C_6$-Alkoxy oder $C_1$-$C_6$-Haloalkoxy bedeuten, unter Einschluss ihrer tautomeren Formen und physiologisch verträglichen Salze.

Eine interessante Gruppe bilden Verbindungen der Formel I, worin $R_3$, $R_4$ und $R_5$ wie unter Formel I definiert sind und $R_1$ und $R_2$ unabhängig voneinander für $C_1$-$C_6$-Alkyl, Allyl, $C_3$-$C_6$-Cycloalkyl oder Phenyl stehen.

Das Fünfringazol ($R_3$) ist somit stets über ein Kohlenstoffatom an das phenolische O-Atom des Aminophenol-Teils des Moleküls gebunden und kann seinerseits unsubstituiert oder substituiert vorliegen. Verbindungen der Formeln I und Ia, worin $R_3$ beispielsweise eine der nachfolgend angegebenen Bedeutungen hat, bilden je nach Definition von $R_3$ eine der folgenden erfindungsgemässen bevorzugten Gruppen a bis f:

| Gruppe | $R_3$ | Typ |
|---|---|---|
| a) | 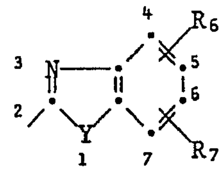 | Benzimidazol-2-yl<br>Benzoxazol-2-yl<br>Benzthiazol-2-yl |
| b) | 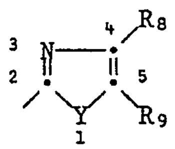 | Imidazol-2-yl<br>Oxazol-2-yl<br>Thiazol-2-yl |
| c) | 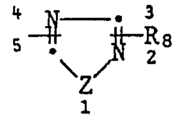 | [1H-1,2,4-Triazol]-5-yl<br>[1,2,4-Oxadiazol]-5-yl<br>[1,2,4-Thiadiazol]-5-yl<br>[1,2,4-Oxadiazol]-3-yl<br>[1,2,4-Thiadiazol]-3-yl |
| d) | 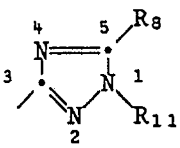 | [1H-1,2,4-Triazol]-3-yl |
| e) | 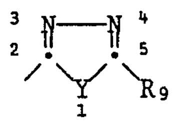 | [1,3,4-Oxadiazol]-2-yl<br>[1,3,4-Thiadiazol]-2-yl<br>[4H-1,2,4-Triazol]-5-yl |
| oder | | |
| f) | 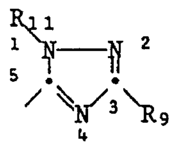 | [1H-1,2,4-Triazol]-5-yl |

wobei

| | |
|---|---|
| Y | für Sauerstoff, Schwefel oder $NR_{10}$ steht; |
| Z | für Sauerstoff, Schwefel oder NH steht; |
| $R_6$ und $R_7$ | unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, $(C_1$-$C_4$-$Alkyl)_2N$, $(C_1$-$C_4$-Alkyl)HN, Halogen, Nitro oder Cyano bedeuten; |
| $R_8$ und $R_9$ | unabhängig voneinander für Wasserstoff, $C_1$-$C_6$-Alkyl, $c_1$-$c_6$-Haloalkyl, $C_1$-$C_6$-Alkoxy, $C_1$-$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen; |
| $R_{10}$ | Wasserstoff oder $C_1$-$C_6$-Alkyl bedeutet; und |
| $R_{11}$ | für $C_1$-$C_6$-Alkyl steht. |

Innerhalb der Gruppen b bis f sind auch diejenigen Verbindungen der Formeln I und Ia interessant, worin Y, Z, $R_{10}$ und $R_{11}$ wie oben definiert sind und $R_8$ und $R_9$ unabhängig voneinander jedoch für unsubstituiertes oder durch Halogen oder $C_1$-$C_3$-Alkyl substituiertes $C_3$-$C_7$-Cycloalkyl, vorzugsweise Cyclopropyl oder Cyclohexyl stehen.

Der Substituent $R_3$ hat somit u.a. folgende Bedeutungen:

a) 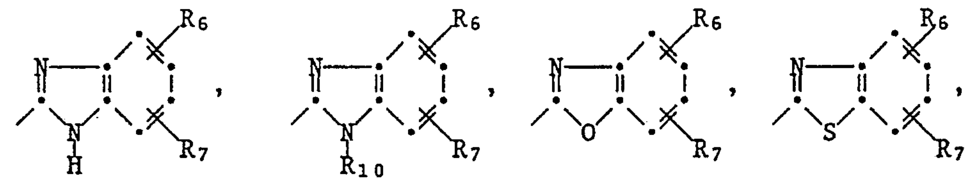

b) 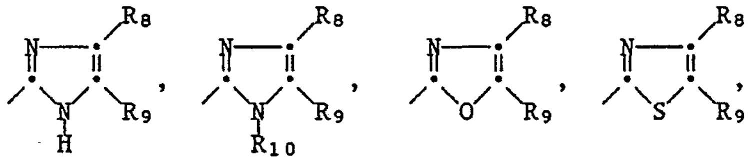

c) 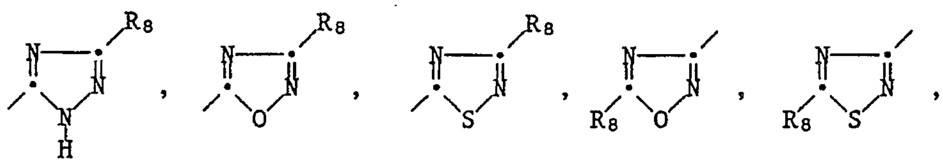

d) 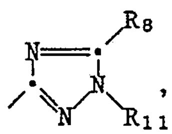

e) 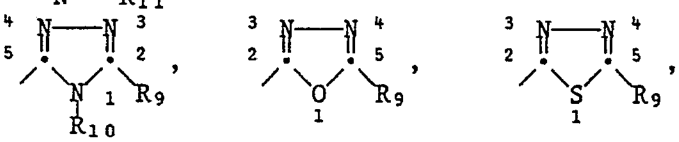

f) 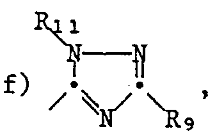

wobei die Substituenten $R_6$ bis $R_{11}$ wie vorstehend definiert sind.

Bevorzugt sind alle Gruppen von Verbindungen der Formeln I und Ia, die formal durch Kombination der Molekülfragmente I' und Ia'

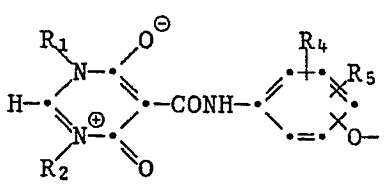 oder 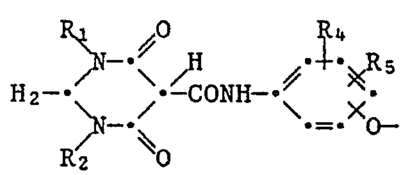

(I') (Ia')

worin $R_1$, $R_2$, $R_4$ und $R_5$ die unter Formel I angegebenen Bedeutungen haben, mit einem unter a bis f genannten Azole $R_3$ gebildet werden. Jede dieser Gruppen ist Bestandteil der vorliegenden Erfindung.

Bevorzugt innerhalb aller genannten Untergruppen sind jene Verbindungen der Formeln I und Ia, worin $R_1$ und $R_2$ unabhängig voneinander für Methyl, Ethyl, Isopropyl, Allyl oder Cyclopropyl stehen und $R_4$ und $R_5$ unabhängig voneinander für Wasserstoff, Methyl, $CF_3$, $OCH_3$, $OCHF_2$oder $OCF_3$stehen.

Innerhalb der Gruppe, worin das Fünfringazol die unter a) angegebene Gruppierung bedeutet sind diejenigen Verbindungen der Formeln I und Ia zusätzlich bevorzugt, worin $R_6$ und $R_7$ unabhängig voneinander für Wasserstoff, Methyl, $CF_3$, Methoxy, Halomethoxy, Fluor, Chlor oder Brom stehen und die übrigen Substituenten wie oben definiert sind.

Innerhalb der Gruppen, worin das Fünfringazol eine der unter b) bis f) angegebenen Gruppierungen bedeutet sind diejenigen Verbindungen der Formeln I und Ia zusätzlich bevorzugt, worin $R_8$ und $R_9$ unabhängig voneinander für Wasserstoff, Methyl, Ethyl, $C_3$-$C_7$-Cycloalkyl, $CF_3$, $C_2F_5$, $C_3F_7$, $CCl_3$, $CHCl_2$, $CH_2Cl$ , $SCH_3$oder Halogen stehen; $R_{10}$ Wasserstoff oder Methyl bedeutet und $R_{11}$ für $C_1$-$C_4$-Alkyl, insbesondere Methyl steht.

Besonders bemerkenswert sind Verbindungen der Formeln I und Ia worin die $OR_3$-Gruppe in para-Stellung an den Phenylrest gebunden ist.

In Formel I ist nur eine der zahlreichen möglichen Betainstrukturen wiedergegeben, weitere isoelektronische Strukturformeln sind z.B.:

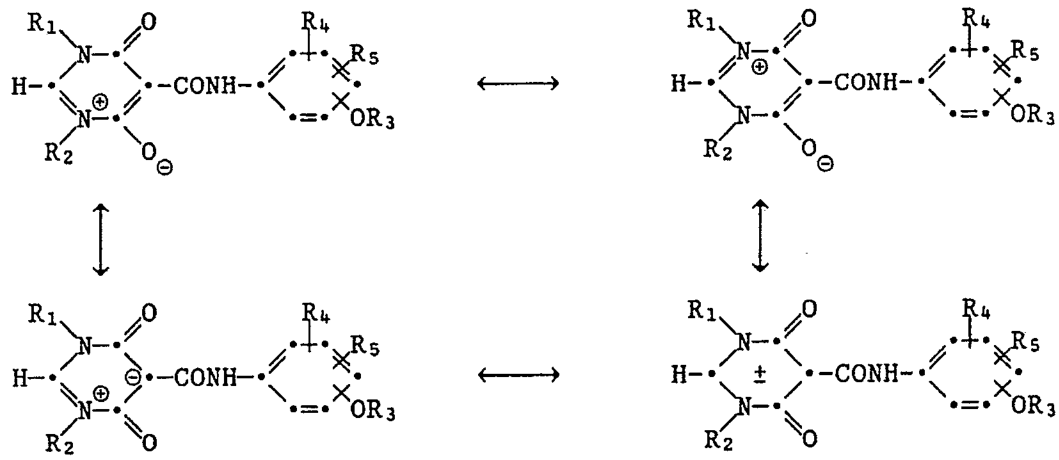

oder sumarisch:

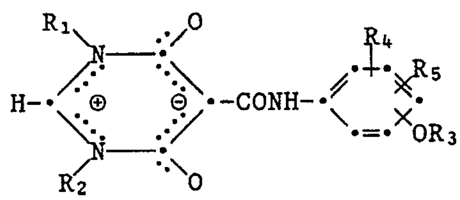

.

Die Verbindungen der Formel Ia können z.B. in folgenden tautomeren Formen vorliegen:

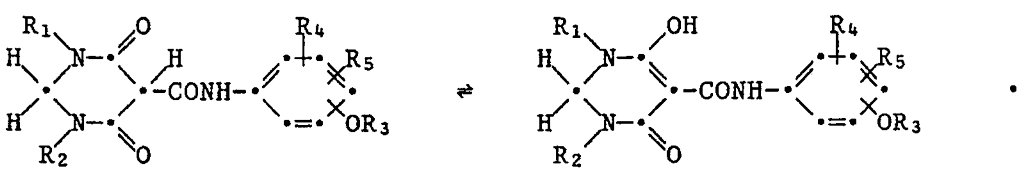

.

Die Verbindungen der Formeln I und Ia sind in allen Formen Bestandteil der vorliegenden Erfindung.

Unter dem Begriff Alkyl selbst oder als Bestandteil eines anderen Substituenten sind im Rahmen vorliegender Erfindung je nach Anzahl der angegebenen Kohlenstoffatome folgende geradkettige und verzweigte Gruppen zu verstehen, z.B.: Methyl, Ethyl, n-Propyl, iso-Propyl, n-Butyl, sek.-Butyl, tert.Butyl, Isobutyl, etc.. Haloalkyl selbst oder als Bestandteile für Haloalkoxy steht für einen einfach bis perhalogenierten Alkylsubstituenten, wie z.B. für $CH_2Cl$ , $CHCl_2$, $CCl_3$, $CH_2F$ , $CHF_2$, $CF_3$, $CH_2Br$ , $CHBr_2$, $CBr_3$, $CH_2J$ , $CJ_3$, $CHClF$ , $CHBrCl$ , $CFBrCl$ , $C_2F_5$, $CH_2CH_2Cl$ , $CHClCH_3$, $C_2Cl_5$, $CHFCHCl_2$, etc., vorzugsweise für $CF_3$. Unter Halogen soll hier und im folgenden Fluor, Chlor, Brom oder Jod, vorzugsweise Fluor, Chlor oder Brom, vor allem jedoch Chlor verstanden werden.

Cycloalkyl selbst oder als Bestandteil eines Substituenten steht je nach Anzahl der angegebenen Kohlenstoffatome für Cyclopropyl, Cyclobutyl, Cyclopentyl, Cyclohexyl, etc. Cyanoalkyl steht für eine Alkylgruppe, worin ein Wasserstoffatom durch CN substituiert ist, vorzugsweise für eine Alkylgruppe, worin

sich die CN-Gruppe am terminalen Kohlenstoffatom befindet.

Unter den Begriff physiologisch verträgliche Salze von Verbindungen der Formel I und Ia fallen die Alkalimetall-, Ammonium- oder Aminsalze, wobei Natrium-, Kalium-, Ammonium- oder Alkylaminsalze, insbesondere Triethylaminsalze, bevorzugt sind. Es sind darunter aber auch die Additionssalze anorganischer und organischer Säuren zu verstehen, die durch Anlagerung einer equivalenten Menge einer salzbildenden Säure an das Basismolekül entstehen.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie z.B. Essigsäure, Trifluoressigsäure, Trichloressigsäure, Propionsäure, Glykolsäure, Thiocyansäure, Milchsäure, Bernsteinsäure, Zitronensäure, Ameisensäure, Benzolsulfonsäure, p-Toluolsulfonsäure, Methansulfonsäure, Salicylsäure, p-Aminosalicylsäure, Phthalsäure, 2-Phenoxybenzoesäure oder 2-Acetoxybenzoesäure.

Die Verbindungen der Formel I und Ia liegen bei Raumtemperatur überwiegend als stabile Feststoffe vor, die einen Schmelzpunkt von ca. 100° bis ca. 300° C aufweisen. Sie besitzen sehr wertvolle anthelminthische Eigenschaften und lassen sich zur kurativen und präventiven Bekämpfung einer Reihe von Wurmerkrankungen bei Warmblütern, insbesondere bei Haus- und Nutztieren, vor allem aus der Klasse der Säugetiere einsetzen.

Besonders bevorzugte Einzelvertreter der Formel I sind:

1 ,3-Dimethyl-5-[4-(6-trifluormethoxy-benzthiazol-2-yloxy)-phenyl-carbamoyl]-4(6)-oxo-6(4)-oxido-(1H,5H)-pyrimidiniumbetain; 1,3-Dimethyl-5-[4-(6-fluor-[5- oder 7-chlor]-benzthiazol-2-yloxy)-phenyl-carbamoyl]-4(6)-oxo-6(4)-oxido-(1H,5H)-pyrimidiniumbetain; 1,3-Dimethyl-5-[4-(5-tert.-butyl-1,3,4-thiadiazol-2-yloxy)-phenyl-carbamoyl]-4(6)-oxo-6(4)-oxido-(1H,5H)-pyrimidiniumbetain; 1,3-Dimethyl-5-[4-(6-chlor-benzthiazol-2-yloxy)-phenyl-carbamoyl]-4(6)-oxo-6(4)-oxido(1H,5H)-pyrimidiniumbetain.

Besonders bevorzugte Vertreter der Formel Ia sind:

1 ,3-Dimethyl-5-[4-(6-trifluormethoxy-benzthiazol-2-yloxy)-phenyl-carbamoyl]-4,6-(1H,3H,5H)-pyrimidindion; 1,3-Dimethyl-5-[4-(6-fluor-[5- oder 7-chlor]-benzthiazol-2-yloxy)-phenyl-carbamoyl]-4,6-(1H,3H,5H)-pyrimidindion; 1 ,3-Dimethyl-5-[4-(5-tert.-butyl-1,3,4-thiadiazol-2-yloxy)-phenyl-carbamoyl]-4,6-(1H,3H,5H)-pyrimidindion; 1,3-Dimethyl-5-[4-(6-chlor-benzthiazol-2-yloxy)-phenyl-carbamoyl]-4,6-(1H,3H,5H)-pyrimidindion.

Die Verbindungen der Formeln I und Ia werden erfindungsgemäss dadurch hergestellt, dass man eine Verbindung der Formel II,

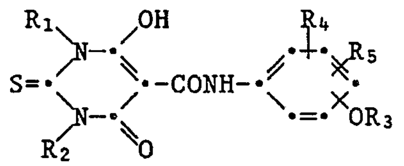

(II)

worin $R_1$, $R_2$, $R_3$, $R_4$ und $R_5$ die unter Formel I und Ia angegebenen Bedeutungen haben entschwefelt. Die Entschwefelung kann z.B. durch Hydrierung, vorzugsweise durch katalytische Hydrierung, vorgenommen werden. In einer bevorzugten Ausführungsform wird die Verbindung der Formel II in einem reaktionsinerten Lösungsmittel oder Lösungsmittelgemisch bei Temperaturen im Bereich von 20 bis 180° C, vorzugsweise 60 bis 120° C, insbesondere bei Rückflusstemperatur mit Trialkylzinnhydrid, Trialkylzinnhalogenid, Trialkylgermaniumhydrid, Trialkylgermaniumhalogenid, Alkylquecksilberhydrid oder Alkylquecksilberhalogenid hydriert, wobei bei der Verwendung eines Halogenids zusätzlich in Gegenwart von $NaBH_4$gearbeitet wird. Alkyl bedeutet in diesem Zusammenhang vorzugsweise $C_1$-$C_6$-Alkyl, insbesondere $C_2$-$C_4$-Alkyl, Halogenid bedeutet insbesondere Chlorid oder Bromid. Besonders geeignet sind die Chloride. Als weiteres geeignetes Hydrierungsmittel ist Tris(trimethylsilyl)silan ($[(CH_3)_3Si]_3SiH$) zu nennen.

Die Hydride und Halogenide werden in mindestens äquimolarer Menge, bezogen auf die Ausgangsverbindung der Formel II, eingesetzt. $NaBH_4$kann ebenfalls äquimolar zugesetzt werden.

Man kann zusätzlich in Anwesenheit eines Radikalinitiators arbeiten, wobei dieser in katalytischen Mengen zugesetzt werden kann. Geeignete Radikalinitiatoren sind z.B. Azoisobutyronitril (AiBN) , Peroxide wie Benzoylperoxid, aber auch UV-Licht oder Wärme. Als geeignete reaktionsinerte Lösungsmittel, allein oder im Gemisch untereinander, kommen z.B. in Frage: Aliphatische und aromatische Kohlenwasserstoff, wie z.B. Pentan, Hexan, Petrolether, Ligroin, Benzol, Toluol, Xylole, usw.; Ether und etherartige Substanzen wie Tetrahydrofuran, Anisol, Dioxan, usw.; halogenierte Kohlenwasserstoffe wie Tetrachlorkohlenstoff, Tetrachlorethylen, Chlorbenzol, etc.

Die Verbindungen I und Ia fallen im allgemeinen nebeneinander an, wobei man durch geeignete Wahl des Lösungsmittels das System so wählen kann, dass z.B. die schwerlöslichen Betaine der Formel I ausfallen und aus dem Reaktionsgemisch z.B. durch Filtration, entfernt werden können, während die löslicheren Verbindungen der Formel Ia aus der Lösung, z.B. durch Einengen oder ausfällen, erhältlich sind. Man kann aber auch ein Gemisch von I und Ia isolieren und anschliessend, z.B. auf Grund ihres unterschiedlichen Lösungsverhaltens, eine Trennung vornehmen, z.B. durch fraktionierte Kristallisation oder durch Säulenchromatographie. Die geschickte Wahl der Lösungsmittel führt aber u.U. auch zu reinen Produkten, d.h. entweder vollständig zu I oder zu Ia. Die Verbindungen der Formel I lassen sich nach üblichen Methoden durch Hydrierung, z.B. mit Raney-Nickel, $NaBH_4$Tributylzinnhydrid, etc., in die Verbindungen der Formel Ia überführen.

Wie bereits eingangs erwähnt, können die Verbindungen der Formel I und Ia auch als Addukte mit Basen oder Säuren vorliegen.

Während Säureadditionssalze bereits eingangs eingehend beschrieben wurden, sind hier noch die anorganischen und organischen Basen zu erwähnen, die als Adduktbildner in Frage kommen. Dies sind z.B. vorzugsweise tertiäre Amine wie Trialkylamine (z.B. Trimethylamin, Triethylamin oder Tripropylamin), Pyridin und Pyridinbasen (z.B. 4-Dimethylaminopyridin, oder 4-Pyrrolidylaminopyridin), Picoline und Lutidine sowie Oxide, Hydroxide, Carbonate und Hydrogencarbonate von Alkali- und Erdalkalimetallen (z.B. CaO, BaO, NaOH , KOH, $KHCO_3$, $NaHCO_3$, $Ca(OH)_2$, $K_2CO_3$oder $Na_2CO_3$), ferner Acetate wie z.B. $CH_3COONa$ oder $CH_3COOK$ . Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100 % der äquimolaren Menge zugesetzt.

In manchen Fällen kann es von Vorteil sein, die Reaktion unter Schutzgasatmosphäre durchzuführen. Geeignete Schutzgase sind z.B. Stickstoff, Helium oder Argon.

Die Umsetzung von II mit Trialkylzinnhydrid, vor allem in Gegenwart eines Radikalstarters, ist besonders bevorzugt.

Die Thiobarbiturate der Formel II sind z.B. aus der Europäischen Offenlegungsschrift EP-192,180 oder dem korrespondierenden Britischen Patent GB-2,171,099B bekannt oder können analog zu den dort beschriebenen Vertretern hergestellt werden.

Die erfindungsgemässen Wirkstoffe der Formel I und Ia können in unterschiedlichen tautomeren Formen vorliegen, nämlich in der Keto- oder Enolform oder in einem Gemisch aus Keto- und Enolform. Die vorliegende Erfindung betrifft sowohl die einzelnen Tautomeren als auch deren Gemische, aber auch die Salze jeder dieser Formen und deren Herstellung.

Das beschriebene Herstellungsverfahren einschliesslich aller Varianten ist ein Bestandteil vorliegender Erfindung.

Wie allgemein bekannt, verursachen unter den bei Warmblütern vorkommenden Endoparasiten namentlich die Helminthen bei den von ihnen heimgesuchten Tieren grosse Schäden. Solche durch Helminthiasen verursachten Schäden können bei chronischem und vor allem bei epidemischem Auftreten der Wurmerkrankungen in Viehherden ein volkswirtschaftlich ins Gewicht fallendes Ausmass annehmen. Sie äussern sich bei den erkrankten Tieren unter anderem in Produktivitäts-Einbussen, geschwächter Widerstandskraft gegenüber weiteren Krankheiten und erhöhter Mortalität. Besonders gefährliche Wurmkrankheiten werden durch im Magen-Darmtrakt und anderen Organen parasitierende Helminthen hervorgerufen und können beispielsweise bei Wiederkäuern wie Rindern, Schafen und Ziegen sowie Pferden, Schweinen, Geflügel, Rotwild, Hunden und Katzen auftreten.

In der vorliegenden Beschreibung werden unter dem Begriff Helminthen insbesondere parasitische Würmer verstanden, die zu den Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.).

Es ist deshalb eine vordringliche Aufgabe, therapeutische Mittel zu entwickeln, die sich zur Bekämpfung von Helminthen in allen ihren Entwicklungsstadien sowie zur Vorbeugung gegen den Befall durch diese Parasiten eignen.

Es sind zwar eine Reihe von Stoffen mit anthelminthischer Wirkung bekannt, die für die Bekämpfung der verschiedenen Helminthen-Species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38, 1425-1426 (1977); Am. J. Vet. Res. 37, 1515-1516 (1976); Am. J. Vet. Res. 38, 807-808 (1977); Am. J. Vet. Res. 38, 1247-1248 (1977)) besitzt nur ein

begrenztes anthelmintisches Wirkungsspektrum bei Wiederkäuern. Seine Wirkung gegen Benzimidazolresistente Nematoden und adulte Leberegel ist völlig unzureichend, wobei vor allem die pathogen wichtigen unreifen Wanderformen der letzteren bei den für das Wirtstier verträglichen Dosierungen nicht angegriffen werden.

Es wurde überraschenderweise gefunden, dass die neuen Verbindungen der Formel I und Ia ein breites Wirkungsspektrum gegen im Tierorganismus, vor allem in Säugetieren, parasitierende Helminthen wie Nematoden, Cestoden und Trematoden besitzen, wobei ihre Wirkung sich vorzugsweise gegen Nematoden (Rundwürmer) richtet.

Als besonderes Merkmal der Verbindungen der Formel I und Ia ist ihre gegenüber Warmblütern überraschend hohe Verträglichkeit hervorzuheben, die sie gegenüber den bekannten Thiobarbitursäurederivaten überlegen macht. Ihre praktische Handhabung bei der Behandlung Wurmerkrankter Tiere wird dadurch ausserordentlich erleichtert, da sie auch in höheren Dosen von den medikierten Tieren symptomlos vertragen werden.

Die erfindungsgemässen neuen Wirkstoffe der Formel I und Ia eignen sich beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren wie Rindern, Schafen, Ziegen, Pferden, Schweinen, Rotwild, Katzen, Hunden und Geflügel. Sie können den Tieren sowohl als Einzeldosis als auch wiederholt verabreicht werden, wobei die einzelnen Gaben je nach Tierart vorzugsweise zwischen 1 und 20 mg pro kg Körpergewicht betragen. Durch eine protrahierte Verabreichung kann in manchen Fällen eine bessere Wirkung erzielt oder mit geringeren Gesamtdosen ausgekommen werden.

Die erfindungsgemäsen Mittel werden hergestellt, indem die Wirkstoffe der Formel I oder Ia mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet: Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nichtionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder -ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I oder Ia nicht-ionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche

synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufig werden sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes oder Phospholipide in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im aliphatischen Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben: "Mc Cutcheon's Detergents and Emulsifiers Annual", MC Publishing Corp., Ridgewood New Jersey, 1980; Sisley and Wood, "Encyclopedia of Surface Active Agents", Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelminthischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I oder Ia direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral oder subcutan durchgeführt werden, wobei die Mittel in Form von Lösungen, Emulsionen, Suspensionen (Drenchs), Pulvern, Tabletten, Boli und Kapseln vorliegen.

Die erfindungsgemässen anthelminthischen Mittel enthalten in der Regel 0,1 bis 99 Gew.-%, insbesondere 0,1 bis 95 Gew.-% Wirkstoff der Formel I, Ia oder Gemische davon, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelminthische Mittel sind ebenfalls ein Bestandteil der vorliegenden Erfindung.

In jedem der erfindungsgemässen Verfahren zur Schädlingsbekämpfung bzw. der erfindungsgemässen Schädlingsbekämpfungsmittel können die Wirkstoffe der Formel I und Ia in allen tautomeren Formen, deren Mischungen oder in Form ihrer Salze eingesetzt werden.

Die Erfindung schliesst auch ein Verfahren zum prophylaktischen Schutz von Tieren gegen parasitäre Helminthen ein, das dadurch gekennzeichnet ist, dass man die Wirkstoffe der Formel I und Ia bzw. die Wirkstofformulierungen als Zusatz zum Futter oder zu den Tränken oder auch in fester oder flüssiger Form oral, durch Injektion oder parenteral den Tieren appliziert.

Die nachfolgenden Beispiele dienen der Illustration der Erfindung, ohne sie einzuschränken.

1. Herstellungsbeispiele

1.1 Herstellung von 1,3-Dimethyl-5-[4-(6-trifluormethoxy-benzthiazol-2-yloxy)-phenylcarbamoyl]-4(6)-oxo-6-(4)-oxido-(1H,5H)-pyrimidiniumbetain

Eine Lösung von 5,3 g (10 mMol) 1,3-Dimethyl-5-[4-(6-trifluormethoxybenzthiazol-2-yloxy)-phenylcarbamoyl]-2-thiobarbitursäure, 126 mg (0,8 mMol) Azoisobutyronitril und 8,8 g (30 mMol) Tributylzinnhydrid werden 3 Stunden in Benzol und unter Stickstoffatmosphäre und unter Rückfluss gerührt. Nach Abkühlen des Reaktionsgemisches auf Raumtemperatur wird das ausgefallene Produkt abfiltriert, mehrfach mit Diisopropylether gewaschen und getrocknet. Man erhält 1,2 g (24 %) der Titelsubstanz. Smp. 230-232 °C.

$^{1}$H-NMR (300 MHz, DMSO $d_6$, TMS): 11,00, s, NH; 9,16, s, H-C(3); 7,40, d, J=9,5, 2H; 7,32, d, J=9,5, 1H; 7,23, 6rs, 1H; 6,92 - 6,81, m, 1H; 6,87, d, J=9,5, 2H; 3,16, s, 2 x $CH_3$.

1.2 Herstellung von 1,3-Dimethyl-5-[4-(6-trifluormethoxy-benzthiazol-2-yloxy)-phenylcarbamoyl]-4,6-(1H,3H,5H)-pyrimidindion

Das gemäss Beispiel 1.1 gewonnene Filtrat wird eingeengt und das ausgefallene Produkt abfiltriert und mehrfach aus Diisopropylether umkristallisiert. Man erhält 2,03 g (46 %) der Titelsubstanz.
Smp. 109-113 °C.
$^{1}$H-NMR (300 MHz, $CDCl_3$, TMS): 18,25, s, OH; 11,91, s, NH; 7,73, d, J=8,0, 2H; 7,59, d, J=6,5, 1H; 7,55, brs, 1H; 7,32, d, J= 8,0, 2H; 7,28 - 7,23, m, 1H; 4,51, s, $H_2$-C(3); 3,01, s, $CH_3$; 2,97, s, $CH_3$.

1.3 Herstellung von 1,3-Dimethyl-5-[4-(5-tert.-butyl-1,3,4-thiadiazol-2-yloxy)-phenylcarbamoyl]-4,6-(1H,3H,5H)-pyrimidindion

Eine Lösung von 5 g (11 mMol) 1,3-Dimethyl-5-[4-(5-tert.-butyl-1,3,4-thiadiazol-2-yloxy)-phenylcarbamoyl]-2-thiobarbitursäure, 9 ml (34 mMol) Tributylzinnhydrid und 90 mg (0,5 mMol) Azoisobutyronitril werden in 200 ml Toluol 5 Stunden unter Rückfluss gerührt, (Stickstoffatmosphäre). Das auf Raumtemperatur abgekühlte Gemisch wird eingeengt und das Rohprodukt mit Hexan/Ether gewaschen, filtriert, getrocknet und aus Essigester umkristallisiert. Man erhält 2,1 g der Titelsubstanz.
Smp. 128-130 °C.
$^{1}$H-NMR (300 MHz, $CDCl_3$, TMS): 18,22, s, OH; 11,88, s, NH; 7,53, d, J=9,5, 2H; 7,18, d, J=9,5, 2H; 4,50, s, $H_2$-C(3); 3,02, s, $CH_3$; 2,96, s, $CH_3$; 1,43, s, 9H.

1.4 Herstellung von 1,3-Dimethyl-5-[4-(5-oder 7-Chlor-6-fluor-benzthiazol-2-yloxy)-phenylcarbamoyl]-4,6-(1H,3H,5H)-pyrimidindion

Eine Suspension von 461 mg (1 mMol) 1,3-Dimethyl-5-[4-(5- oder 7-Chlor-6-fluorbenzthiazol-2-yloxy)-phenylcarbamoyl]-4(6)-oxo-6(4)-oxido-(1H,5H)pyrimidiniumbetain, 68 mg (2 mMol) $NaBH_4$in 10 ml Ethanol wird unter Stickstoff 1 Stunde bei Raumtemperatur gerührt. Das auf 0 °C abgekühlte Reaktionsgemisch wird

mit 2N HCl angesäuert, dann mit gesättigter $NaHCO_3$-Lösung neutralisiert und mit Essigester extrahiert. Die organische Phase wird mit gesättigter $NaHCO_3$-Lösung und dann mit wässriger gesättigter NaCl -Lösung gewaschen, auf $MgSO_4$getrocknet und eingeengt. Man erhält, nach Umkristallisation aus Essigester, 253 mg der Titelsubstanz.

Smp. 179-183 °C.

$^1$H-NMR (300 MHz, $CDCl_3$, TMS): 18,25, s, OH; 11,93, s, NH; 7,80 - 7,16, m, 6H; 4,52, s, $H_2$-C(3); 3,03, s, $CH_3$; 2,98, s, $CH_3$.

Analog zu den beschriebenen Vorgehensweisen lassen sich auch die in den folgenden Tabellen genannten Vertreter der Formeln I und Ia herstellen.

Tabelle 1: Verbindungen der Formel

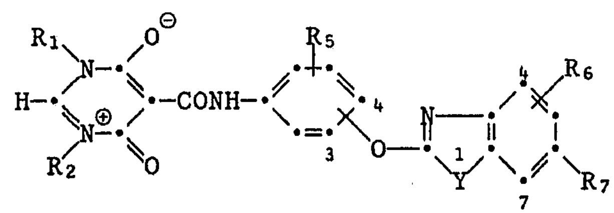

| Verb. Nr. | $R_1$ | $R_2$ | $R_5$ | $R_6$ | $R_7$ | Y | Position der Heterocyclyl-oxygruppe | physikalische Daten Smp. [°C] $^1$H-NMR (300 MHz, DMSO $d_6$, TMS) |
|---|---|---|---|---|---|---|---|---|
| 1.1 | $CH_3$ | $CH_3$ | H | H | $OCF_3$ | S | 4 | 230-232° |
| 1.2 | $CH_3$ | $CH_3$ | H | 5- oder 7-Cl | F | S | 4 | 225-237° |
| 1.3 | $CH_3$ | $CH_3$ | H | H | Cl | S | 4 | 227-229° |
| 1.4 | $CH_3$ | $CH_3$ | H | H | $CF_3$ | S | 4 | 244-246° |
| 1.5 | $CH_3$ | $CH_3$ | H | H | F | S | 4 | 258-262° |
| 1.6 | $CH_3$ | $CH_3$ | H | H | H | O | 4 | |
| 1.7 | $CH_3$ | $CH_3$ | H | H | Cl | O | 4 | |
| 1.8 | $CH_3$ | $CH_3$ | H | H | Cl | NH | 4 | |
| 1.9 | $CH_3$ | $CH_3$ | $3-OCH_3$ | H | H | S | 4 | |
| 1.10 | $CH_3$ | $CH_3$ | $4-OCH_3$ | H | Cl | S | 3 | |
| 1.11 | $CH_3$ | $C_2H_5$ | H | H | $OCF_3$ | S | 4 | 210-212° |
| 1.12 | $CH_3$ | $C_2H_5$ | H | 5- oder 7-Cl | F | S | 4 | |

Fortsetzung von Tabelle 1:

| Verb. Nr. | $R_1$ | $R_2$ | $R_5$ | $R_6$ | $R_7$ | Y | Position der Heterocyclyl-oxygruppe | physikalische Daten Smp. [°C] $^1$H-NMR (300 MHz, DMSO $d_6$, TMS) |
|---|---|---|---|---|---|---|---|---|
| 1.13 | $CH_3$ | $C_2H_5$ | H | H | Cl | S | 4 | |
| 1.14 | $CH_3$ | $CH_2CH=CH_2$ | H | H | $OCF_3$ | S | 4 | 195-197° |
| 1.15 | $CH_3$ | $CH_2CH=CH_2$ | H | H | Cl | S | 4 | |
| 1.16 | $CH_3$ | $CH_2CH=CH_2$ | H | H | Cl | O | 4 | |
| 1.17 | $CH_3$ | $C_2H_5$ | 3-$OCH_3$ | H | $CF_3$ | S | 4 | |
| 1.18 | $CH_3$ | $CH_3$ | H | H | Cl | $N(CH_3)$ | 4 | |
| 1.19 | $CH_3$ | $CH_3$ | H | H | Cl | $N(CH_3)$ | 3 | |
| 1.20 | $CH_3$ | $CH_3$ | H | H | H | NH | 4 | |
| 1.21 | $CH_3$ | $CH_3$ | H | H | $OC_2H_5$ | S | 4 | 285° |
| 1.22 | $CH_3$ | $CH_3$ | H | H | $OCHF_2$ | S | 4 | 191-193° |
| 1.23 | $CH_3$ | $CH_3$ | H | H | $OCH_3$ | S | 4 | 235-239° |
| 1.24 | $CH_3$ | $CH_3$ | H | 5-$CF_3$ | H | S | 4 | 300-302° |
| 1.25 | $CH_3$ | $CH_3$ | H | 5-$OCH_3$ | H | S | 4 | 292-295° |
| 1.26 | $CH_3$ | $CH_3$ | H | H | $C_2H_5$ | S | 4 | 292-234° |
| 1.27 | $CH_3$ | $CH_3$ | 2-$C_3H_7$i | H | $OCF_3$ | S | 4 | |
| 1.28 | $CH_3$ | $CH_3$ | 2,6-$(CH_3)_2$ | H | $OCF_3$ | S | 4 | |
| 1.29 | $CH_3$ | $CH_3$ | 2-$CH_3$ | H | $OCF_3$ | S | 4 | |
| 1.30 | $CH_3$ | $CH_3$ | 3-$OC_2H_5$ | H | $OCF_3$ | S | 4 | |

Fortsetzung von Tabelle 1:

| Verb. Nr. | $R_1$ | $R_2$ | $R_5$ | $R_6$ | $R_7$ | Y | Position der Heterocyclyl-oxygruppe | physikalische Daten Smp. [°C] $^1$H-NMR (300 MHz, DMSO $d_6$, TMS) |
|---|---|---|---|---|---|---|---|---|
| 1.31 | $CH_3$ | $CH_3$ | 4-$OCH_3$ | H | $OCF_3$ | S | 3 | |
| 1.32 | $CH_3$ | $CH_3$ | H | H | $OCF_3$ | S | 3 | |
| 1.33 | $CH_3$ | $CH_3$ | 4-$CH_3$ | H | $OCF_3$ | S | 2 | |
| 1.34 | $CH_3$ | $CH_3$ | H | H | $N(CH_3)$ | S | 4 | |
| 1.35 | $CH_3$ | $CH_3$ | H | H | $SCF_3$ | S | 4 | |
| 1.36 | $CH_3$ | $CH_3$ | H | H | $S(O)CF_3$ | S | 4 | |
| 1.37 | $CH_3$ | $CH_3$ | H | H | $S(O_2)CF_3$ | S | 4 | |
| 1.38 | $CH_3$ | $CH_3$ | H | 5-$N(CH_3)_2$ | H | S | 4 | |
| 1.39 | $CH_3$ | $CH_3$ | H | 5-$SCF_3$ | H | S | 4 | |
| 1.40 | $CH_3$ | $CH_3$ | H | 5-$S(O)CF_3$ | H | S | 4 | |
| 1.41 | $CH_3$ | $CH_3$ | H | 5-$S(O)_2CF_3$ | H | S | 4 | |

Tabelle 2: Verbindungen der Formel

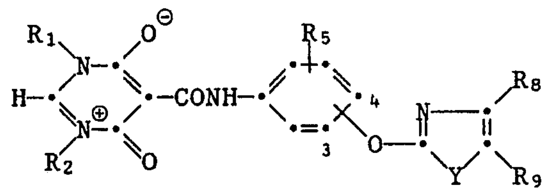

| Verb. Nr. | $R_1$ | $R_2$ | $R_5$ | $R_8$ | $R_9$ | Y | Position der Heterocyclyl-oxygruppe | physikalische Daten Smp. [°C] $^1$H-NMR (300 MHz, DMSO $d_6$, TMS) |
|---|---|---|---|---|---|---|---|---|
| 2.1 | $CH_3$ | $CH_3$ | H | Cl | Cl | NH | 4 | |
| 2.2 | $CH_3$ | $CH_3$ | H | Cl | Cl | $N(CH_3)$ | 4 | |
| 2.3 | $CH_3$ | $CH_3$ | H | Cl | Cl | NH | 3 | |
| 2.4 | $CH_3$ | $CH_3$ | H | Cl | Cl | $N(CH_3)$ | 3 | |
| 2.5 | $CH_3$ | $CH_3$ | H | H | H | O | 4 | |
| 2.6 | $CH_3$ | $C_2H_5$ | H | H | H | O | 4 | |
| 2.7 | $CH_3$ | $CH_3$ | H | H | H | S | 4 | |
| 2.8 | $CH_3$ | $C_2H_5$ | H | H | H | S | 3 | |

Tabelle 3: Verbindungen der Formel

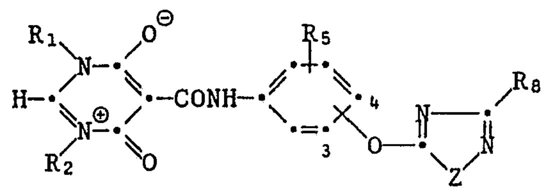

| Verb. Nr. | $R_1$ | $R_2$ | $R_5$ | $R_8$ | Z | Position der Heterocyclyl-oxygruppe | physikalische Daten Smp. [°C] $^1$H-NMR (300 MHz, DMSO $d_6$, TMS) |
|---|---|---|---|---|---|---|---|
| 3.1 | $CH_3$ | $CH_3$ | H | $CF_3$ | O | 4 | |
| 3.2 | $CH_3$ | $CH_3$ | H | $CF_3$ | O | 3 | |
| 3.3 | $CH_3$ | $CH_3$ | H | $CF_3$ | S | 4 | |
| 3.4 | $CH_3$ | $CH_3$ | H | $CF_3$ | S | 3 | |
| 3.5 | $CH_3$ | $CH_3$ | H | $CF_3$ | NH | 4 | |
| 3.6 | $CH_3$ | $C_2H_5$ | H | $CF_3$ | S | 4 | |
| 3.7 | $CH_3$ | $CH_3$ | H | Cl | S | 4 | |

Tabelle 4: Verbindungen der Formel

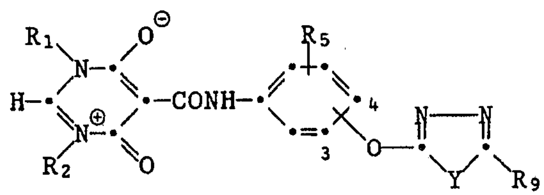

| Verb. Nr. | $R_1$ | $R_2$ | $R_5$ | $R_9$ | Y | Position der Heterocyclyl-oxygruppe | physikalische Daten Smp. [°C] $^1$H-NMR (300 MHz, DMSO $d_6$, TMS) |
|---|---|---|---|---|---|---|---|
| 4.1 | $CH_3$ | $CH_3$ | H | tert.-butyl | O | 4 | |
| 4.2 | $CH_3$ | $CH_3$ | H | tert.-butyl | S | 4 | |
| 4.3 | $CH_3$ | $CH_3$ | H | tert.-butyl | NH | 4 | |
| 4.4 | $CH_3$ | $CH_3$ | H | tert.-butyl | O | 3 | |
| 4.5 | $CH_3$ | $CH_3$ | H | tert.-butyl | S | 3 | |
| 4.6 | $CH_3$ | $CH_3$ | H | $CH_3$ | S | 4 | |
| 4.7 | $CH_3$ | $C_2H_5$ | H | $CH_3$ | O | 4 | |

Tabelle 5: Verbindungen der Formel

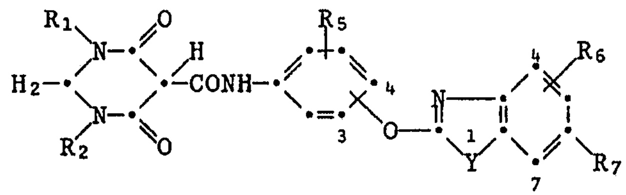

| Verb. Nr. | $R_1$ | $R_2$ | $R_5$ | $R_6$ | $R_7$ | Y | Position der Heterocyclyloxygruppe | physikalische Daten Smp. [°C] $^1$H-NMR (300 MHz, $CDCl_3$, TMS) |
|---|---|---|---|---|---|---|---|---|
| 5.1 | $CH_3$ | $CH_3$ | H | H | $OCF_3$ | S | 4 | 109-113° |
| 5.2 | $CH_3$ | $CH_3$ | H | 5- oder 7-Cl | F | S | 4 | 179-183° |
| 5.3 | $CH_3$ | $CH_3$ | H | H | Cl | S | 4 | 18.25,s,OH; 11.94,s,NH; 4.52,s,$H_2$-C(3) |
| 5.4 | $CH_3$ | $CH_3$ | H | H | $CF_3$ | S | 4 | 128-130° |
| 5.5 | $CH_3$ | $CH_3$ | H | H | F | S | 4 | 173-174° |
| 5.6 | $CH_3$ | $CH_3$ | H | H | H | O | 4 | |
| 5.7 | $CH_3$ | $CH_3$ | H | H | Cl | O | 4 | |
| 5.8 | $CH_3$ | $CH_3$ | H | H | Cl | NH | 4 | |
| 5.9 | $CH_3$ | $CH_3$ | 3-$OCH_3$ | H | H | S | 4 | |
| 5.10 | $CH_3$ | $CH_3$ | 4-$OCH_3$ | H | Cl | S | 3 | |
| 5.11 | $CH_3$ | $C_2H_5$ | H | H | $OCF_3$ | S | 4 | 95-97° |

Fortsetzung von Tabelle 5:

| Verb. Nr. | $R_1$ | $R_2$ | $R_5$ | $R_6$ | $R_7$ | Y | Position der Heterocyclyl-oxygruppe | physikalische Daten Smp. [°C] $^1$H-NMR (300 MHz, $CDCl_3$, TMS) |
|---|---|---|---|---|---|---|---|---|
| 5.12 | $CH_3$ | $C_2H_5$ | H | 5- oder 7-Cl | F | S | 4 | |
| 5.13 | $CH_3$ | $C_2H_5$ | H | H | Cl | S | 4 | |
| 5.14 | $CH_3$ | $CH_2CH=CH_2$ | H | H | $OCF_3$ | S | 4 | 136-137° |
| 5.15 | $CH_3$ | $CH_2CH=CH_2$ | H | H | Cl | S | 4 | |
| 5.16 | $CH_3$ | $CH_2CH=CH_2$ | H | H | Cl | O | 4 | |
| 5.17 | $CH_3$ | $C_2H_5$ | 3-$OCH_3$ | H | $CF_3$ | S | 4 | |
| 5.18 | $CH_3$ | $CH_3$ | H | H | Cl | N($CH_3$) | 4 | |
| 5.19 | $CH_3$ | $CH_3$ | H | H | Cl | N($CH_3$) | 3 | |
| 5.20 | $CH_3$ | $CH_3$ | H | H | H | NH | 4 | |
| 5.21 | $CH_3$ | $CH_3$ | H | 5-Cl | H | S | 4 | 181-183° |
| 5.22 | $CH_3$ | $CH_3$ | H | $OC_2H_5$ | H | S | 4 | 149-151° |
| 5.23 | $CH_3$ | $CH_3$ | H | $OCHF_2$ | H | S | 4 | 161-163° |
| 5.24 | $CH_3$ | $CH_3$ | H | $OCH_3$ | H | S | 4 | 140-142° |
| 5.25 | $CH_3$ | $CH_3$ | 5-$CF_3$ | H | H | S | 4 | 159-160° |
| 5.26 | $CH_3$ | $CH_3$ | 5-$OCH_3$ | H | H | S | 4 | 127-129° |
| 5.27 | $CH_3$ | $CH_3$ | H | H | H | S | 4 | 151-153° |
| 5.28 | $CH_3$ | $CH_3$ | H | $C_2H_5$ | H | S | 4 | 151-153° |
| 5.29 | $CH_3$ | $CH_3$ | 2-$C_3H_7$i | H | $OCF_3$ | S | 4 | |

Fortsetzung von Tabelle 5:

| Verb. Nr. | $R_1$ | $R_2$ | $R_5$ | $R_6$ | $R_7$ | Y | Position der Heterocyclyloxygruppe | physikalische Daten Smp. [°C] $^1$H-NMR (300 MHz, $CDCl_3$, TMS) |
|---|---|---|---|---|---|---|---|---|
| 5.30 | $CH_3$ | $CH_3$ | 2,6-$(CH_3)_2$ | H | $OCF_3$ | S | 4 | |
| 5.31 | $CH_3$ | $CH_3$ | 2-$CH_3$ | H | $OCF_3$ | S | 4 | |
| 5.32 | $CH_3$ | $CH_3$ | 3-$OC_2H_5$ | H | $OCF_3$ | S | 4 | |
| 5.33 | $CH_3$ | $CH_3$ | 4-$OCH_3$ | H | $OCF_3$ | S | 3 | |
| 5.34 | $CH_3$ | $CH_3$ | H | H | $OCF_3$ | S | 3 | |
| 5.35 | $CH_3$ | $CH_3$ | 4-$CH_3$ | H | $OCF_3$ | S | 2 | |
| 5.36 | $CH_3$ | $CH_3$ | H | H | $N(CH_3)_2$ | S | 4 | |
| 5.37 | $CH_3$ | $CH_3$ | H | H | $SCF_3$ | S | 4 | |
| 5.38 | $CH_3$ | $CH_3$ | H | H | $S(O)CF_3$ | S | 4 | |
| 5.39 | $CH_3$ | $CH_3$ | H | H | $S(O)_2)CF_3$ | S | 4 | |
| 5.40 | $CH_3$ | $CH_3$ | H | 5-$N(CH_3)_2$ | H | S | 4 | |
| 5.41 | $CH_3$ | $CH_3$ | H | 5-$SCF_3$ | H | S | 4 | |
| 5.42 | $CH_3$ | $CH_3$ | H | 5-$S(O)CF_3$ | H | S | 4 | |
| 5.43 | $CH_3$ | $CH_3$ | H | 5-$S(O)_2CF_3$ | H | S | 4 | |

Tabelle 6: Verbindungen der Formel

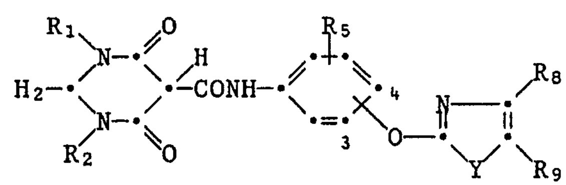

| Verb. Nr. | $R_1$ | $R_2$ | $R_5$ | $R_8$ | $R_9$ | Y | Position der Heterocyclyl-oxygruppe | physikalische Daten Smp. [°C] $^1$H-NMR (300 MHz, $CDCl_3$, TMS) |
|---|---|---|---|---|---|---|---|---|
| 6.1 | $CH_3$ | $CH_3$ | H | Cl | Cl | NH | 4 | |
| 6.2 | $CH_3$ | $CH_3$ | H | Cl | Cl | $N(CH_3)$ | 4 | |
| 6.3 | $CH_3$ | $CH_3$ | H | Cl | Cl | NH | 3 | |
| 6.4 | $CH_3$ | $CH_3$ | H | Cl | Cl | $N(CH_3)$ | 3 | |
| 6.5 | $CH_3$ | $CH_3$ | H | H | H | O | 4 | |
| 6.6 | $CH_3$ | $C_2H_5$ | H | H | H | O | 4 | |
| 6.7 | $CH_3$ | $CH_3$ | H | H | H | S | 4 | |
| 6.8 | $CH_3$ | $C_2H_5$ | H | H | H | S | 3 | |

<u>Tabelle 7:</u> Verbindungen der Formel

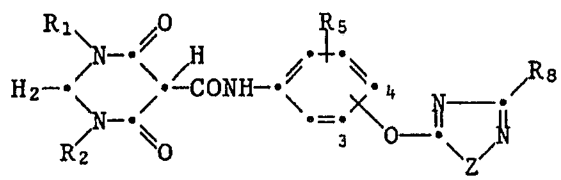

| Verb. Nr. | $R_1$ | $R_2$ | $R_5$ | $R_8$ | Z | Position der Heterocyclyl-oxygruppe | physikalische Daten Smp. [°C] $^1$H-NMR (300 MHz, $CDCl_3$, TMS) |
|---|---|---|---|---|---|---|---|
| 7.1 | $CH_3$ | $CH_3$ | H | $CF_3$ | O | 4 | |
| 7.2 | $CH_3$ | $CH_3$ | H | $CF_3$ | O | 3 | |
| 7.3 | $CH_3$ | $CH_3$ | H | $CF_3$ | S | 4 | |
| 7.4 | $CH_3$ | $CH_3$ | H | $CF_3$ | S | 3 | |
| 7.5 | $CH_3$ | $CH_3$ | H | $CF_3$ | NH | 4 | |
| 7.6 | $CH_3$ | $C_2H_5$ | H | $CF_3$ | S | 4 | |
| 7.7 | $CH_3$ | $CH_3$ | H | Cl | S | 4 | |

Tabelle 8: Verbindungen der Formel

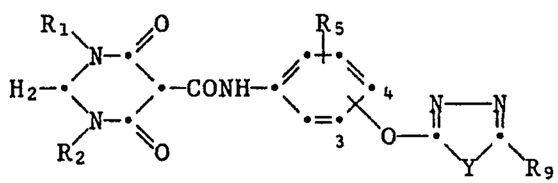

| Verb. Nr. | $R_1$ | $R_2$ | $R_5$ | $R_9$ | Y | Position der Heterocyclyl-oxygruppe | physikalische Daten Smp. [°C] $^1$H-NMR (300 MHz, $CDCl_3$, TMS) |
|---|---|---|---|---|---|---|---|
| 8.1 | $CH_3$ | $CH_3$ | H | tert.-butyl | O | 4 | |
| 8.2 | $CH_3$ | $CH_3$ | H | tert.-butyl | S | 4 | 128-130° |
| 8.3 | $CH_3$ | $CH_3$ | H | tert.-butyl | NH | 4 | |
| 8.4 | $CH_3$ | $CH_3$ | H | tert.-butyl | O | 3 | |
| 8.5 | $CH_3$ | $CH_3$ | H | tert.-butyl | S | 3 | |
| 8.6 | $CH_3$ | $CH_3$ | H | $CH_3$ | S | 4 | |
| 8.7 | $CH_3$ | $C_2H_5$ | H | $CH_3$ | O | 4 | |

2. Formulierungsbeispiele (% = Gewichtsprozent)

| 2.1. Emulsions-Konzentrate | a) | b) | c) |
|---|---|---|---|
| Wirkstoff aus den Tabellen | 25 % | 40% | 50% |
| Ca-Dodecylbenzolsulfonat | 5 % | 8% | 6% |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus diesen Konzentraten können durch Verdünnen mit Wasser Emulsionen jeder gewünschten Konzentration hergestellt werden.

| 2.2. Emulsions-Konzentrate | a) | b) | c) |
|---|---|---|---|
| Wirkstoff aus den Tabellen | 10 % | 8 % | 60 % |
| Octylphenolpolyethylenglykolether (4-5 Mol Ethylenoxid) | 3 % | 3 % | 2 % |
| Ca-Dodecylbenzolsulfonat | 3 % | 4 % | 4 % |
| Ricinusölpolyglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesen Konzentraten können durch Verdünnen mit Wasser Emulsionen jeder gewünschten Konzentration hergestellt werden.

| 2.3. Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein

Suspensions-Konzentrat, aus welchem durch Verdünnen mit Wasser Suspensionen jeder gewünschten Konzentration hergestellt werden können.

| 2.4. In Wasser dispergierbare Pulvermischungen | a) | b) | c) |
|---|---|---|---|
| Wirkstoff aus den Tabellen | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.5. Stäubemittel | a) | b) |
|---|---|---|
| Wirkstoff der Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff und Vermahlen des Gemisches erhält man gebrauchsfertige Stäubemittel.

| 2.6. Granulat | a) | b) |
|---|---|---|
| Wirkstoff der Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

| 2.7. Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.8. Granulat

| | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.9. Tabletten bzw. Boli

| | | |
|---|---|---|
| I | Wirkstoff aus den Tabellen | 33,00 % |
| | Methylcellulose | 0,80 % |
| | Kieselsäure hochdispers | 0,80 % |
| | Maisstärke | 8,40 % |
| II | Milchzucker krist. | 22,50 % |
| | Maisstärke | 17,00 % |
| | mikrokrist. Cellulose | 16,50 % |
| | Magnesiumstearat | 1,00 % |

I Methylcellulose wird in Wasser eingerührt. Nachdem das Material gequollen ist, wird Kieselsäure eingerührt und das Gemisch homogen suspendiert. Wirkstoff und Maisstärke werden gemischt. In diese Mischung wird die wässrige Suspension eingearbeitet und zu einem Teig geknetet. Die so erhaltene Masse wird durch ein 12 M-Sieb granuliert und getrocknet.

II Alle 4 Hilfsstoffe werden gut gemischt.

III Die gemäss I und II erhaltenen Vormischungen werden gemischt und zu Tabletten oder Boli verpresst.

2.10. Injektabiles

A. Oeliges Vehikel (langsame Freisetzung)

| | |
|---|---|
| Ein Wirkstoff aus den Tabellen | 0,1-1,0 g |
| Erdnussöl | ad 100 ml |

| | |
|---|---|
| Ein Wirkstoff aus den Tabellen | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

B. Wassermischbare Lösung (mittlere Freisetzungsgeschwindigkeit)

| | |
|---|---|
| Ein Wirkstoff aus den Tabellen | 0,1-1,0 g |
| 4-Hydroxymethyl-1,3-dioxolan (Glycerol Formal) | 40 g |
| 1,2-Propandiol | ad 100 ml |

| | |
|---|---|
| Ein Wirkstoff aus den Tabellen | 0,1-1,0 g |
| Glycerindimethylketal | 40 g |
| 1,2-Propandiol | ad 100 ml |

Herstellung: Der Wirkstoff wird in einem Teil des Lösungsmittels unter Rühren gelöst, auf das Sollvolumen aufgefüllt und durch ein geeignetes Membranfilter mit 0.22 µm sterilfiltriert.

C. Wässriges Solubilisat (rasche Freisetzung)

| | |
|---|---|
| Ein Wirkstoff aus den Tabellen | 0,1-1,0 g |
| Polyäthoxyliertes Ricinusöl (40 Aethylenoxideinheiten) | 10 g |
| 1,2-Propandiol | 20 g |
| Benzylalkohol | 1 g |
| Aqua ad injekt. | ad 100 ml |

| | |
|---|---|
| Ein Wirkstoff aus den Tabellen | 0,1-1,0 g |
| Polyäthoxyliertes Sorbitanmonooleat (20 Aethylenoxideinheiten) | 8 g |
| 4-Hydroxymethyl-1,3-dioxolan (Glycerol Formal) | 20 g |
| Benzylalkohol | 1 g |
| Aqua ad injekt. | ad 100 ml |

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 µm Porendurchmesser.

Die wässrigen Systeme können bevorzugterweise auch für die orale und/oder intraruminale Applikation eingesetzt werden.

3. Biologische Beispiele

Die anthelminthische Wirksamkeit wird anhand folgender Versuche demonstriert:

3.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf

wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

In diesem Test wird mit Verbindungen der Formeln I und Ia eine starke Reduzierung des Nematodenbefalls erzielt. So wird beispielsweise beim Einsatz von 20 mg Wirksubstanz pro kg Körpergewicht mit den nachfolgenden Verbindungen eine Reduzierung des Nematodenbefalls von ca. 90 % bewirkt: 1.1, 5.1 und 5.2. Dieses Ergebnis wird bei einzelnen Verbindungen auch noch mit weiter herabgesetzter Dosis, beispielsweise mit 10 mg Wirksubstanz pro kg Körpergewicht oder noch geringeren Mengen an Wirksubstanz, erreicht.

3.2. Versuch an mit Cestoden wie Moniezia benedeni infizierten Schafen

Der Wirkstoff wird in Form einer Suspension mit einer Magensonde oder durch Pansen-Injektion Schafen verabreicht, die vorher mit Cestoden wie Moniezia benedeni artifiziell infiziert wurden. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken Wirkstoffe aus den Tabellen, wie beispielsweise die Verbindungen Nr. 1.1, 5.1 und 5.2. bei Dosen von weniger als 20 mg/kg Körpergewicht eine ca. 90%ige Reduktion des Cestodenbefalls.

3.3. Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica artifiziell infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen Wirkstoffe aus den Tabellen bei Dosen von weniger als 20 mg/kg Körpergewicht eine ausgeprägte Wirksamkeit gegen Fasciola hepatica.

## Ansprüche

1. Verbindung der allgemeinen Formel I

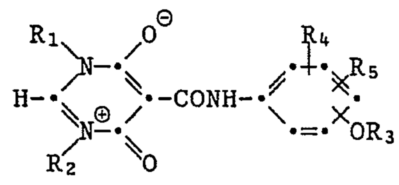

(I)

oder in ihrer hydrierten Form mit der Formel (Ia)

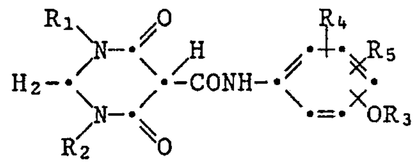

(Ia)

worin

$R_1$ und $R_2$ unabhängig voneinander für $C_1$-$C_6$-Alkyl, Allyl, $C_3$-$C_6$-Cycloalkyl, Benzyl oder Phenyl stehen; und

$R_3$ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol; und $R_4$ und $R_5$ unabhängig voneinander Wasserstoff, $C_1$-$C_6$-Alkyl, $C_1$-$C_6$-Haloalkyl, $C_1$-$C_6$-Alkoxy oder $C_1$-$C_6$-Haloalkoxy bedeuten, unter Einschluss ihrer tautomeren Formen und physiologisch verträglichen Salze.

**2.** Verbindung nach Anspruch 1, dadurch gekennzeichnet, dass $R_1$ und $R_2$ unabhängig voneinander für $C_1$-$C_6$-Alkyl, Allyl, $C_3$-$C_6$-Cycloalkyl oder Phenyl stehen und $R_3$, $R_4$ und $R_5$ die in Anspruch 1 angegebenen Bedeutungen haben.

**3.** Verbindung der Formel I oder Ia nach Anspruch 2, dadurch gekennzeichnet, dass $R_1$ und $R_2$ unabhängig voneinander für $C_1$-$C_6$-Alkyl, Allyl, $C_3$-$C_6$-Cycloalkyl oder Phenyl stehen; $R_4$ und $R_5$ unabhängig voneinander für Wasserstoff, $C_1$-$C_6$-Alkyl, $C_1$-$C_6$-Haloalkyl, $C_1$-$C_6$-Alkoxy oder $C_1$-$C_6$-Haloalkoxy stehen und $R_3$ einen der folgenden Heterocyclen repräsentiert ausgewählt aus der Gruppe

a) 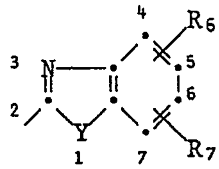

Benzimidazol-2-yl
Benzoxazol-2-yl
Benzthiazol-2-yl

b) 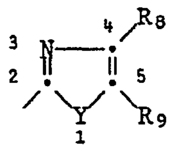

Imidazol-2-yl
Oxazol-2-yl
Thiazol-2-yl

c) 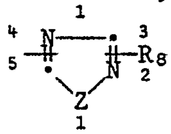

[1H-1,2,4-Triazol]-5-yl
[1,2,4-Oxadiazol]-5-yl
[1,2,4-Thiadiazol]-5-yl
[1,2,4-Oxadiazol]-3-yl
[1,2,4-Thiadiazol]-3-yl

d) 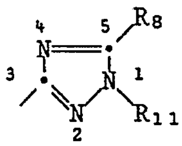

[1H-1,2,4-Triazol]-3-yl

e) 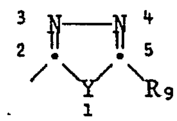

[1,3,4-Oxadiazol]-2-yl
[1,3,4-Thiadiazol]-2-yl
[4H-1,2,4-Triazol]-5-yl

und

f) 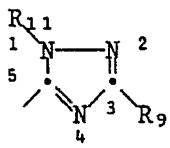

[1H-1,2,4-Triazol]-5-yl

wobei

| | |
|---|---|
| Y | für Sauerstoff, Schwefel oder $NR_{10}$steht; |
| Z | für Sauerstoff, Schwefel oder NH steht; |
| $R_6$ und $R_7$ | unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, $(C_1$-$C_4$-Alkyl$)_2$N, ($C_1$-$C_4$-Alkyl)HN, Halogen, Nitro oder Cyano bedeuten; |
| $R_8$ und $R_9$ | unabhängig voneinander für Wasserstoff, $C_1$-$C_6$-Alkyl, $C_1$-$C_6$-Haloalkyl, $C_1$-$C_6$-Alkoxy, $C_1$-$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen; |
| $R_{10}$ | Wasserstoff oder $C_1$-$C_6$-Alkyl bedeutet; und |
| $R_{11}$ | für $C_1$-$C_6$-Alkyl steht. |

**4.** Verbindung der Formel I nach Anspruch 3, worin $R_1$, $R_2$, $R_3$, $R_4$, $R_5$, Y, Z, sowie $R_8$, $R_9$, $R_{10}$ und $R_{11}$ die in Anspruch 3 angegebenen Bedeutungen haben.

**5.** Verbindung der Formel I oder Ia nach Anspruch 3, dadurch gekennzeichnet, dass innerhalb der Gruppen b) bis f) $R_8$ und $R_9$ unabhängig voneinander für unsubstituiertes oder durch Halogen oder $C_1$-$C_3$-Alkyl substituiertes $C_3$-$C_7$-Cycloalkyl stehen und die übrigen Substituenten wie in Anspruch 2 definiert sind.

**6.** Verbindung der Formel I oder Ia nach Anspruch 3, dadurch gekennzeichnet, dass $R_3$ eine der folgenden Gruppen repräsentiert

a) 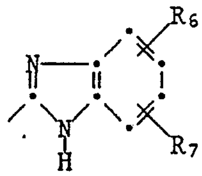 , 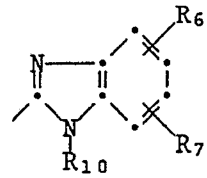 , 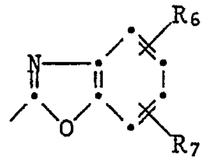 , 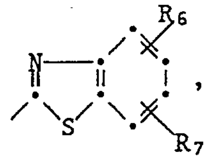 ,

b) 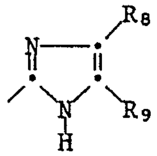 , 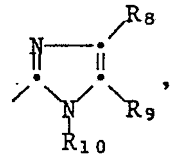 , 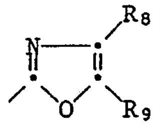 , 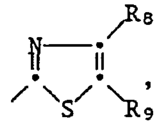 ,

c) 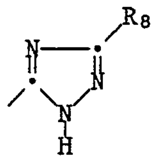 , 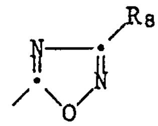 , 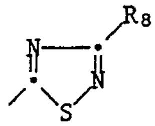 , 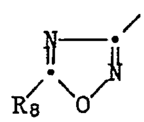 , 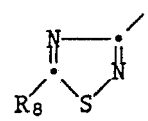 ,

d) 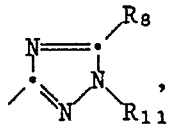 ,

e) 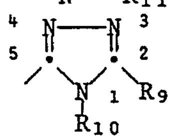 , 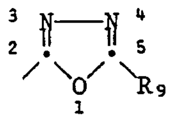 , 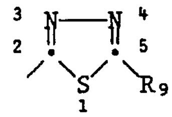 ,

f) 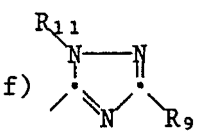 ,

wobei

| | |
|---|---|
| Y | für Sauerstoff, Schwefel oder $NR_{10}$steht; |
| Z | für Sauerstoff, Schwefel oder NH steht; |
| $R_6$ und $R_7$ | unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, Halogen, Nitro oder Cyano bedeuten; |
| $R_8$ und $R_9$ | unabhängig voneinander für Wasserstoff, $C_1$-$C_6$-Alkyl, $C_1$-$C_6$-Haloalkyl, $C_1$-$C_6$-Alkoxy, $C_1$-$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen. |

7. Verbindung der Formel I oder Ia nach einem der Ansprüche 1 bis 6, dadurch gekennzeichnet, dass $R_1$ und $R_2$ unabhängig voneinander für Methyl, Ethyl, Isopropyl, Allyl oder Cyclopropyl stehen, $R_4$ und $R_5$ unabhängig voneinander für Wasserstoff, Methyl, $CF_3$, $OCH_3$, $OCHF_2$oder $OCF_3$stehen und die übrigen Substituenten wie vorgängig definiert sind.

8. Eine Verbindung der Formel I nach Anspruch 2, ausgewählt aus der Reihe 1,3-Dimethyl-5-[4-(6-trifluormethoxy-benzthiazol-2-yloxy)-phenyl-carbamoyl]-4(6)-oxo-6(4)-oxido-(1H,5H)-pyrimidiniumbetain; 1,3-Dimethyl-5-[4-(6-fluor-[5- oder 7-chlor]-benzthiazol-2-yloxy)-phenyl-carbamoyl]-4(6)-oxo-6(4)-oxido-(1H,5H)-pyrimidiniumbetain; 1,3-Dimethyl-5-[4-(5-tert.butyl-1,3,4-thiadiazol-2-yloxy)-phenyl-carbamoyl]-4(6)-oxo-6(4)-oxido-(1H,5H)-pyrimidiniumbetain; 1,3-Dimethyl-5-[4-(6-chlor-benzthiazol-2-yloxy)-phenyl-carbamoyl]-4(6)-oxo-6(4)-oxido-(1H,5H)-pyrimidiniumbetain.

9. Eine Verbindung der Formel Ia nach Anspruch 2 ausgewählt aus der Reihe 1,3-Dimethyl-5-[4(6-trifluormethoxy-benzthiazol-2-yloxy)-phenyl-carbamoyl]-4,6-(1H,3H,5H)-pyrimidindion; 1,3-Dimethyl-5-[4-(6-fluor-[5- oder 7-chlor]-benzthiazol-2-yloxy)-phenyl-carbamoyl]-4,6-(1H,3H,5H)-pyrimidindion; 1,3-Dimethyl-5-[4-(5-tert.-butyl-1,3,4-thiadiazol-2-yloxy)-phenyl-carbamoyl]-4,6-(1H,3H,5H)-pyrimidindion; 1,3-Dimethyl-5-[4-(6-chlor-benzthiazol-2-yloxy)-phenyl-carbamoyl]-4,6-(1H,3H,5H)-pyrimidindion.

**10.** Verfahren zur Herstellung einer Verbindung mit der Formel I

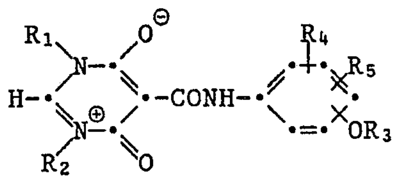

(I)

oder in ihrer hydrierten Form mit der Formel Ia

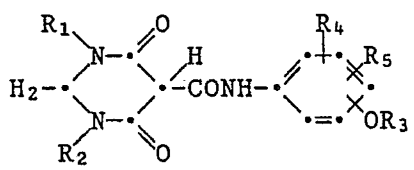

(Ia)

worin
$R_1$ und $R_2$ unabhängig voneinander für $C_1$-$C_6$-Alkyl, Allyl, $C_3$-$C_6$-Cycloalkyl, Benzyl oder Phenyl stehen; und $R_3$ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol; und $R_4$ und $R_5$ unabhängig voneinander Wasserstoff, $C_1$-$C_6$-Alkyl, $C_1$-$C_6$-Haloalkyl, $C_1$-$C_6$-Alkoxy oder $C_1$-$C_6$-Haloalkoxy bedeuten, unter Einschluss ihrer tantomeren Formen und physiologisch verträglichen Salze, dadurch gekennzeichnet, dass man eine Verbindung der Formel II,

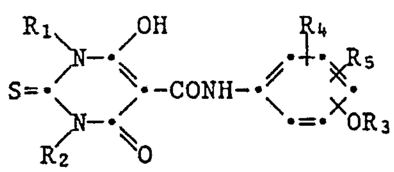

(II)

worin $R_1$, $R_2$, $R_3$, $R_4$ und $R_5$ die unter Formel I und Ia angegebenen Bedeutungen haben, entschwefelt.

**11.** Verfahren nach Anspruch 10, dadurch gekennzeichnet, dass man die Entschwefelung durch katalytische Hydrierung in einem reaktionsinerten Lösungsmittel oder Lösungsmittelgemisch bei Temperaturen in einem Bereioch von 20 bis 180 °C, vorzugsweise 60 bis 120 °C, insbesondere bei Rückflusstemperatur vornimmt.

**12.** Verfahren nach Anspruch 11, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$arbeitet.

**13.** Verfahren nach Anspruch 12, dadurch gekennzeichnet, dass man in Gegenwart eines Radikalinitiators arbeitet.

**14.** Anthelminthisches Mittel, dadurch gekennzeichnet, dass es als aktive Komponente mindestens eine Verbindung der Formel I oder Ia, ein Tautomeres oder ein Salz davon gemäss Anspruch 1 bis 9 neben üblichen Träger- und weiteren Hilfsstoffen enthält.

15. Verbindung der Formel I oder Ia nach einem der Ansprüche 1 bis 9 zur Verwendung in einem Verfahren zur Bekämpfung von Wurmerkrankungen bei Haus- und Nutztieren.

16. Verbindung der Formel I oder Ia nach einem der Ansprüche 1 bis 9 zur Herstellung eines veterinärmedizinischen Pharmazeutikums.

Patentansprüche (für die Vertragsstaaten GR und ES)

1. Verfahren zur Herstellung eines Wirkstoffs mit der Formel I

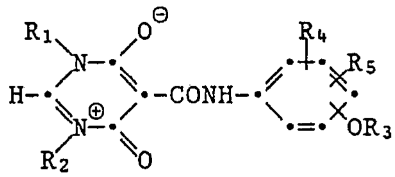

(I)

oder in ihrer hydrierten Form mit der Formel Ia

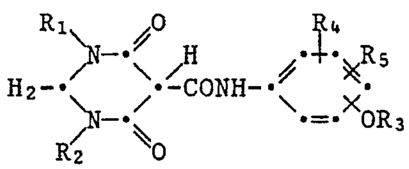

(Ia)

worin
$R_1$ und $R_2$ unabhängig voneinander für $C_1$-$C_6$-Alkyl, Allyl, $C_3$-$C_6$-Cycloalkyl, Benzyl oder Phenyl stehen; und $R_3$ für ein unsubstituiertes oder substituiertes, über Kohlenstoff gebundenes, heteroaromatisches Fünfringazol steht, ausgewählt aus der Reihe Benzimidazol, Benzoxazol, Benzthiazol, Imidazol, Oxazol, Thiazol, Oxadiazol, Thiadiazol, und Triazol; und $R_4$ und $R_5$ unabhängig voneinander Wasserstoff, $C_1$-$C_6$-Alkyl, $C_1$-$C_6$-Haloalkyl, $C_1$-$C_6$-Alkoxy oder $C_1$-$C_6$-Haloalkoxy bedeuten, unter Einschluss ihrer tantomeren Formen und physiologisch verträglichen Salze, dadurch gekennzeichnet, dass man eine Verbindung der Formel II,

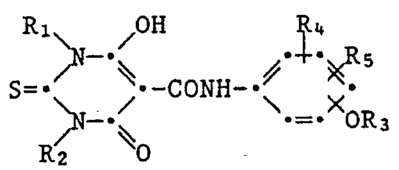

(II)

worin $R_1$, $R_2$, $R_3$, $R_4$ und $R_5$ die unter Formel I und Ia angegebenen Bedeutungen hat entschwefelt.

2. Verfahren nach Anspruch 1, dadurch gekennzeichnet, dass man die Entschwefelung durch katalytische Hydrierung in einem reaktionsinerten Lösungsmittel oder Lösungsmittelgemisch bei Temperaturen in einem Bereioch von 20 bis 180°C, vorzugsweise 60 bis 120°C, insbesondere bei Rückflusstemperatur vornimmt.

3. Verfahren nach Anspruch 2, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$ arbeitet.

4. Verfahren nach Anspruch 3, dadurch gekennzeichnet, dass man in Gegenwart eines Radikalinitiators arbeitet.

5. Verfahren nach einem der Ansprüche 1 bis 4, dadurch gekennzeichnet, dass in Formel I und Ia $R_1$ und $R_2$ unabhängig voneinander für $C_1$-$C_6$-Alkyl, Allyl, $C_3$-$C_6$-Cycloalkyl oder Phenyl stehen und $R_3$, $R_4$ und $R_5$ die in Anspruch 1 angegebenen Bedeutungen haben.

6. Verfahren nach Anspruch 5, dadurch gekennzeichnet, dass in Formel I und Ia $R_1$ und $R_2$ unabhängig voneinander für $C_1$-$C_6$-Alkyl, Allyl, $C_3$-$C_6$-Cycloalkyl oder Phenyl stehen; $R_4$ und $R_5$ unabhängig voneinander für Wasserstoff, $C_1$-$C_6$-Alkyl, $C_1$-$C_6$-Haloalkyl, $C_1$-$C_6$-Alkoxy oder $C_1$-$C_6$-Haloalkoxy stehen und $R_3$ einen der folgenden Heterocyclen repräsentiert ausgewählt aus der Gruppe

a) 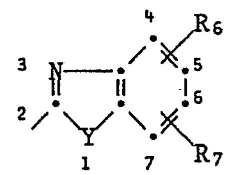 Benzimidazol-2-yl
Benzoxazol-2-yl
Benzthiazol-2-yl

b) 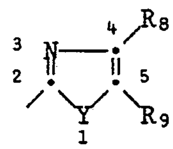 Imidazol-2-yl
Oxazol-2-yl
Thiazol-2-yl

c) 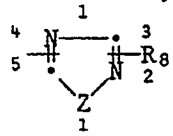 [1H-1,2,4-Triazol]-5-yl
[1,2,4-Oxadiazol]-5-yl
[1,2,4-Thiadiazol]-5-yl
[1,2,4-Oxadiazol]-3-yl
[1,2,4-Thiadiazol]-3-yl

d) 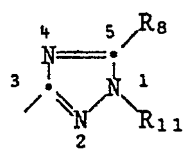 [1H-1,2,4-Triazol]-3-yl

e) 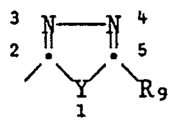 [1,3,4-Oxadiazol]-2-yl
[1,3,4-Thiadiazol]-2-yl
[4H-1,2,4-Triazol]-5-yl

und

f) 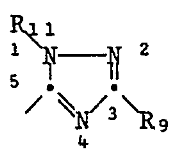 [1H-1,2,4-Triazol]-5-yl

wobei

| | |
|---|---|
| Y | für Sauerstoff, Schwefel oder $NR_{10}$steht; |
| Z | für Sauerstoff, Schwefel oder NH steht; |
| $R_6$ und $R_7$ | unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, $(C_1$-$C_4$-$Alkyl)_2N$, ($C_1$-$C_4$-Alkyl)HN, Halogen, Nitro oder Cyano bedeuten; |
| $R_8$ und $R_9$ | unabhängig voneinander für Wasserstoff, $C_1$-$C_6$-Alkyl, $C_1$-$C_6$-Haloalkyl, $C_1$-$C_6$-Alkoxy, $C_1$-$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen; |
| $R_{10}$ | Wasserstoff oder $C_1$-$C_6$-Alkyl bedeutet; und |
| $R_{11}$ | für $C_1$-$C_6$-Alkyl steht. |

7. Verfahren nach Anspruch 6, dadurch gekennzeichnet, dass in Formel I $R_1$, $R_2$, $R_3$, $R_4$, $R_5$, Y, Z, $R_8$, $R_9$, $R_{10}$ und $R_{11}$ wie in Anspruch 6 definiert sind.

8. Verfahren nach Anspruch 6, dadurch gekennzeichnet, dass in Formel I und Ia innerhalb der Gruppen b) bis f) $R_8$ und $R_9$ unabhängig voneinander für unsubstituiertes oder durch Halogen oder $C_1$-$C_3$-Alkyl substituiertes $C_3$-$C_7$-Cycloalkyl stehen und die übrigen Substituenten wie in Anspruch 5 definiert sind.

9. Verfahren nach Anspruch 6, dadurch gekennzeichnet, dass in Formel I und Ia $R_3$ eine der folgenden Gruppen repräsentiert

a) 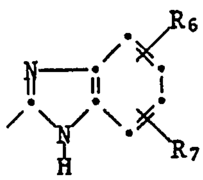 , 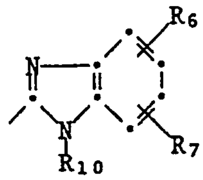 , 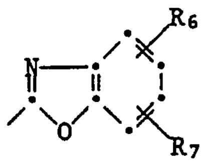 , 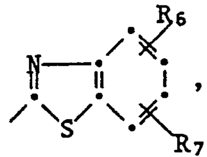 ,

b) 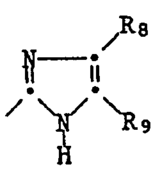 , 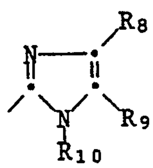 , 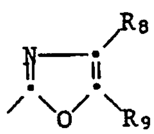 , 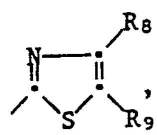 ,

c) 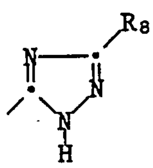 , 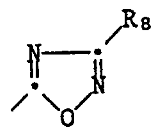 , 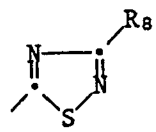 , 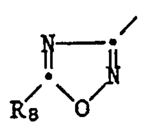 , 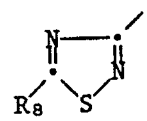 ,

d) 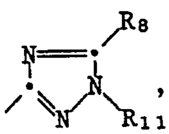 ,

e) 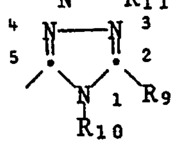 , 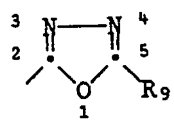 , 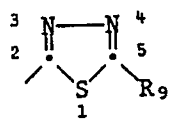 ,

f) 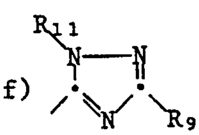 ,

wobei

| | |
|---|---|
| Y | für Sauerstoff, Schwefel oder $NR_{10}$steht; |
| Z | für Sauerstoff, Schwefel oder NH steht; |
| $R_6$ und $R_7$ | unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, Halogen, Nitro oder Cyano bedeuten; |
| $R_8$ und $R_9$ | unabhängig voneinander für Wasserstoff, $C_1$-$C_6$-Alkyl, $C_1$-$C_6$-Haloalkyl, $C_1$-$C_6$-Alkoxy, $C_1$-$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen. |

10. Verfahren nach einem der Ansprüche 1 bis 8, dadurch gekennzeichnet, dass in Formel I und Ia $R_1$ und $R_2$ unabhängig voneinander für Methyl, Ethyl, Isopropyl, Allyl oder Cyclopropyl stehen, $R_4$ und $R_5$ unabhängig voneinander für Wasserstoff, Methyl, $CF_3$, $OCH_3$, $OCHF_2$oder $OCF_3$stehen und die übrigen

Substituenten wie vorgängig definiert sind.

11. Verfahren nach Anspruch 5, dadurch gekennzeichnet, dass es sich bei der Verbindung der Formel I um eine Substanz handelt, ausgewählt aus der Reihe 1,3-Dimethyl-5-[4-(6-trifluormethoxy-benzthiazol-2-yloxy)-phenyl-carbamoyl]-4(6)-oxo-6(4)oxido-(1H,5H)-pyrimidiniumbetain; 1,3-Dimethyl-5-[4-(6-fluor-[5- oder 7-chlor]-benzthiazol-2-yloxy)-phenyl-carbamoyl]-4(6)-oxo-6(4)-oxido-(1H,5H)-pyrimidiniumbetain; 1,3-Dimethyl-5-[4-(5-tert.butyl-1,3,4-thiadiazol-2-yloxy)-phenyl-carbamoyl]-4(6)-oxo-6(4)oxido-(1H,5H)-pyrimidiniumbetain; 1,3-Dimethyl-5-[4-(6-chlor-benzthiazol-2-yloxy)-phenyl-carbamoyl]-4(6)-oxo-6(4)-oxido-(1H,5H)-pyrimidiniumbetain.

12. Verfahren nach Anspruch 5, dadurch gekennzeichnet, dass es sich bei der Verbindung der Formel Ia um eine Substanz handelt, ausgewählt aus der Reihe 1,3-Dimethyl-5-[4-(6-trifluormethoxy-benzthiazol-2-yloxy)-phenyl-carbamoyl]-4,6-(1H,3H,5H)-pyrimidindion; 1,3-Dimethyl-5-[4-(6-fluor-[5- oder 7-chlor]-benzthiazol-2-yloxy)-phenyl-carbamoyl]-4,6-(1H,3H,5H)-pyrimidindion; 1 ,3-Dimethyl-5-[4-(5-tert.-butyl-1,3,4-thiadiazol-2-yloxy)-phenyl-carbamoyl]-4,6-(1H,3H,5H)-pyrimidindion; 1,3-Dimethyl-5-[4-(6-chlor-benzthiazol-2-yloxy)-phenyl-carbamoyl]-4,6-(1H,3H,5H)-pyrimidindion.

13. Verfahren zur Bekämpfung parasitärer Helminthen, dadurch gekennzeichnet, dass man einem Tier eine anthelminthisch wirksame Menge einer Verbindung der Formel I oder Ia gemäss Anspruch 1 bis 12 verabreicht.

14. Verwendung einer Verbindung der Formel I oder Ia gemäss Anspruch 1 bis 12 zur Bekämpfung von parasitären Helminthen.